(19) Europäisches Patentamt
European Patent Office
Office européen des brevets

(11) Numéro de publication: **0 133 061**
**A1**

(12) **DEMANDE DE BREVET EUROPEEN**

(21) Numéro de dépôt: **84401254.2**

(22) Date de dépôt: **18.06.84**

(51) Int. Cl.[4]: **C 07 D 233/64**
A 61 K 31/415

(30) Priorité: **01.07.83 FR 8310974**

(43) Date de publication de la demande: **13.02.85 Bulletin 85/7**

(84) Etats contractants désignés: **AT BE CH DE FR GB IT LI LU NL SE**

(71) Demandeur: **SYNTHELABO**
**58, rue de la Glacière**
**F-75621 Paris Cedex 13(FR)**

(72) Inventeur: **Kaplan, Jean-Pierre**
**20, rue Arnoux**
**F-92340 Bourg-la-Reine(FR)**

(74) Mandataire: **Ludwig, Jacques et al,**
**SYNTHELABO Service Brevets 58, rue de la Glacière**
**F-75621 Paris Cedex 13(FR)**

(54) **Diphénylazométhines portant un radical imidazolyle, leur préparation et leur application en thérapeutique.**

(57) Diphénylazomèthines répondant à la formule

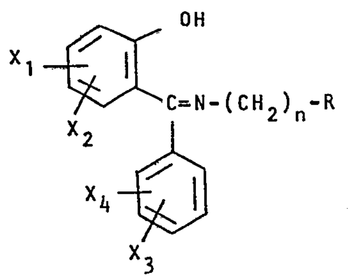

(I)

dans laquelle
$X_1$, $X_2$, $X_3$ et $X_4$ représentent chacun, indépendamment l'un de l'autre, un atome d'hydrogène ou d'halogène ou un groupe nitro, cyano, hydroxy, méthoxy, alkyle en $C_1$-$C_3$, allyle ou phényle, et
soit R représente un radical 1*H*-imidazolyle-2 et n est égal à 1,
soit R représente un radical 1*H*-imidazolyle-4 et n est égal à 1 ou 2.
Application en thérapeutique.

EP 0 133 061 A1

Croydon Printing Company Ltd.

DIPHENYLAZOMETHINES PORTANT UN RADICAL IMIDAZOLYLE, LEUR PREPARATION ET LEUR APPLICATION EN THERAPEUTIQUE.

La présente invention concerne des diphénylazométhines portant un radical imidazolyle, leur préparation et leur application en thérapeutique.

Les composés de l'invention répondent à la formule

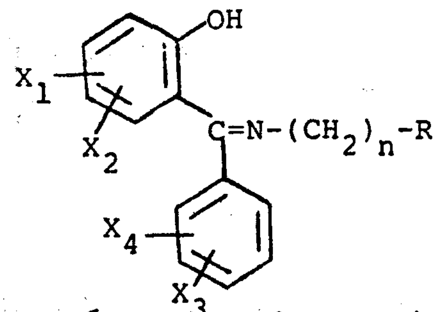

(I)

dans laquelle

$X_1$, $X_2$, $X_3$ et $X_4$ représentent chacun, indépendamment l'un de l'autre, un atome d'hydrogène ou d'halogène ou un groupe nitro, cyano, hydroxy, méthoxy, alkyle en $C_1$-$C_3$, allyle ou phényle, et

soit R représente un radical 1<u>H</u>-imidazolyle-2 et n est égal à 1,

soit R représente un radical 1<u>H</u>-imidazolyle-4 et n est égal à 1 ou 2.

Selon l'invention on peut préparer les composés de l'invention selon le schéma réactionnel suivant :

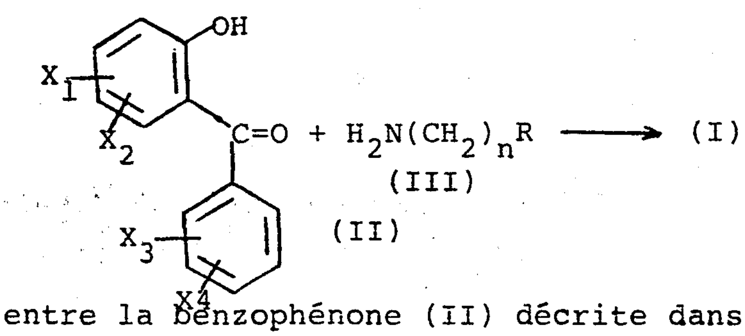

(II) (III) (I)

La réaction entre la benzophénone (II) décrite dans l'un des brevets de la Demanderesse 75 24065, 76 21922 ou 81 21559, et le composé (III), éventuellement sous forme de chlorhydrate, est effectuée dans un solvant tel que le méthanol, l'éthanol ou le toluène, éventuellement, en présence d'une base telle que le bicarbonate de sodium, le carbonate de sodium, le méthylate ou l'éthylate de sodium, à une température de 20 à 110°C.

Les composés (III) sont préparés à partir de composés décrits dans la littérature selon des méthodes décrites dans la littérature.
Les microanalyses et les spectres IR et RMN confirment la structure des composés.

EXEMPLE 1 Chloro-4 [(chloro-2 phényl){[(1H-imidazolyl-4) méthyl]imino}méthyl]-2 phénol.

[$X_1$=4-Cl, $X_2$=H, $X_3$=2-Cl, n=1, R=1H-imidazolyle-4]

Dans un erlenmeyer on introduit 2,67 g (0,009 mole) de (chloro-5 hydroxy-2 phényl)(chloro-2 phényl) méthanone, 2,21 g (0,013 mole) de dichlorhydrate d'imidazolyl-4 méthylamine 2,2 g (0,026 mole) de Na $HCO_3$ et 20 ml d'éthanol absolu. On porte ce mélange à la température du reflux, pendant 24 heures . On évapore le mélange réactionnel à siccité, on reprend le résidu avec de l'eau et du chlorure de méthylène. On filtre l'insoluble et le lave plusieurs fois à l'éther. On obtient un solide jaune. On lave la phase organique à l'eau, la sèche sur $MgSO_4$ et évapore. On obtient un solide jaune identique au précédent.

F=228-230°C.

EXEMPLE2 Chloro-4 [(bromo-2 phényl){[(1H-imidazolyl-4)-2 éthyl]-imino}méthyl]-2 phénol.

[$X_1$=4-Cl, $X_2$=H, $X_3$=2-Br, n=2, R=1H-imidazolyle-4]

Dans un ballon de 500 ml, on introduit 10,3 g de (chloro-5 hydroxy-2 phényl)(bromo-2 phényl) méthanone et 250 ml de méthanol puis on ajoute 6,15 g de chlorhydrate d'histamine et 3,6 g de méthylate de sodium. On évapore le mélange à siccité, on ajoute 250 ml d'éthanol et on évapore à nouveau à siccité. On reprend le résidu par du chloroforme, on lave la phase chloroformique à l'eau, on laisse décanter le mélange et on sèche la phase organique séparée sur $MgSO_4$. On filtre le solide sur fritté. On évapore le filtrat à siccité et on

fait cristalliser le produit dans de l'éther de pétrole. Après recristallisation on le sèche au dessicateur.

F=178,4°C.

Les composés de l'invention préparés à titre d'exemples sont rassemblés dans le tableau suivant.

Tableau

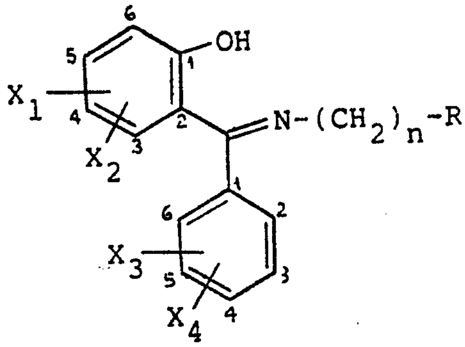

(I)

| Composé | $X_1$ | $X_2$ | $X_3$ | $X_4$ | n | R(*) | F(°C) |
|---|---|---|---|---|---|---|---|
| 1 | 4-Cl | H | 2-Br | H | 2 | (4) | 178,4 |
| 2 | 4-F | H | 4-Cl | H | 2 | (4) | 176,7 |
| 3 | 4-Cl | H | 2-Cl | H | 1 | (2) | 113-114 |
| 4 | 4-Cl | H | 2-Cl | H | 1 | (4) | 228-230 |
| 5 | 4-Cl | $6-CH_3$ | 4-Cl | H | 1 | (4) | 179-180 |
| 6 | 4-Cl | H | 2-Cl | H | 2 | (4) | 168-168 |
| 7 | 4-Cl | H | H | H | 1 | (4) | 167-168 |
| 8 | 4-Cl | H | 2-Cl | 4-Cl | 1 | (4) | 212-213 |
| 9 | 4-Cl | H | $2-CH_3$ | $5-CH_3$ | 1 | (4) | 128 |
| 10 | 6-Cl | 4-Cl | 2-Cl | H | 1 | (4) | 224-225 |
| 11 | $4-NO_2$ | H | H | H | 1 | (4) | 162-163 |
| 12 | 4-Cl | H | 2-Br | H | 1 | (4) | 230-231 |
| 13 | 4-Cl | H | 2-F | H | 1 | (4) | 202-203 |
| 14 | $4-CH_3$ | H | $2-CH_3$ | $5-CH_3$ | 1 | (4) | 183-184 |
| 15 | 4-Br | H | 2-Cl | H | 1 | (4) | 240-241 |
| 16 | $6-CH_3$ | 4-Cl | H | H | 1 | (4) | 179-180 |
| 17 | H | H | H | H | 1 | (4) | 154-155 |
| 18 | 4-Cl | H | $4-CH_3$ | H | 1 | (4) | 202-203 |
| 19 | 4-Cl | H | $2-CH_3$ | H | 1 | (4) | 166-167 |
| 20 | 4-F | H | 4-Cl | H | 1 | (4) | 147-148 |
| 21 | 4-Br | H | 2-Cl | 4-Cl | 1 | (4) | 212-213 |

(*) : (2) représente 1H-imidazolyle-2

(4) représente 1H-imidazolyle-4

Tableau (suite)

| Composé | $X_1$ | $X_2$ | $X_3$ | $X_4$ | n | R(*) | F(°C) |
|---|---|---|---|---|---|---|---|
| 22 | 4-$CH_3$ | H | H | H | 1 | (4) | 170-171 |
| 23 | 4-Cl | H | 4-Cl | H | 1 | (4) | 189-190 |
| 24 | 6-$CH_2CH_2CH_3$ | 4-Cl | 4-Cl | H | 1 | (4) | 178-180 |
| 25 | 4-$CH_3$ | H | 2-Cl | H | 1 | (4) | 194-195 |
| 26 | 5-$CH_3$ | 4-$CH_3$ | 2-Cl | H | 1 | (4) | 148-149 |
| 27 | 5-$CH_3$ | 4-Cl | 2-Cl | H | 1 | (4) | 190-191 |
| 28 | 5-$CH_3$ | 4-Cl | H | H | 1 | (4) | 163-164 |
| 29 | 6-Cl | 4-Cl | H | H | 1 | (4) | 228-229 |
| 30 | 6-$CH_2CH{=}CH_2$ | 4-Cl | H | H | 1 | (4) | 177-178 |
| 31 | H | H | 3-Cl | 4-Cl | 1 | (4) | 164-165 |
| 32 | 5-Cl | 4-Cl | H | H | 1 | (4) | 190-191 |
| 33 | 4-Cl | H | 3-Cl | H | 1 | (4) | 141-142 |
| 34 | 6-$CH_3$ | 4-Cl | 3-Cl | 4-Cl | 1 | (4) | 174-175 |
| 35 | 6-$C_2H_5$ | 4-Cl | 4-$C_2H_5$ | H | 1 | (4) | 140-142 |
| 36 | 6-$CH_2CH{=}CH_2$ | 4-Cl | 2-Cl | H | 1 | (4) | 93-95 |
| 37 | 4-$C_6H_5$ | H | H | H | 1 | (4) | 219-220 |
| 38 | 4-Cl | 3-Cl | H | H | 1 | (4) | 165-166 |
| 39 | 6-Cl | 5-Cl | H | H | 1 | (4) | 222-223 |
| 40 | 6-Cl | 5-Cl | 2-F | H | 1 | (4) | 217-218 |
| 41 | H | H | 2-Cl | H | 1 | (4) | 158-159 |
| 42 | 6-Cl | 3-Cl | H | H | 1 | (4) | 133-134 |
| 43 | 5-Cl | 4-Cl | 2-Cl | H | 1 | (4) | 207-208 |
| 44 | 6-$C_6H_5$ | H | H | H | 1 | (4) | 238-240 |
| 45 | 5-Cl | 3-Cl | H | H | 1 | (4) | 183-184 |
| 46 | 4-$C_6H_5$ | H | 2-F | H | 1 | (4) | 206-207 |
| 47 | 6-CN | H | H | H | 1 | (4) | 194-195 |
| 48 | 6-$C_6H_5$ | H | 2-Cl | H | 1 | (4) | 181-182 |
| 49 | 4-$C_6H_5$ | H | 2-Cl | H | 1 | (4) | 244-245 |
| 50 | 6-Cl | 5-Cl | 2-Cl | H | 1 | (4) | 238-239 |
| 51 | H | H | 2-OH | H | 1 | (4) | 244-245 |

(*) : (2) représente 1H-imidazolyle-2

(4) représente 1H-imidazolyle-4

Tableau (fin)

| Composé | $X_1$ | $X_2$ | $X_3$ | $X_4$ | n | R(*) | F(°C) |
|---|---|---|---|---|---|---|---|
| 52 | 4-Cl | H | H | H | 2 | (4) | 148-149 |
| 53 | 4-Cl | H | 2-F | H | 2 | (4) | 133-134 |
| 54 | 6-$CH_3$ | 4-Cl | 4-Cl | H | 2 | (4) | 139-140 |
| 55 | 4-Cl | H | 4-$CH_3$ | H | 2 | (4) | 159-160 |
| 56 | 4-$CH_3$ | H | H | H | 2 | (4) | 200-201 |
| 57 | 5-$CH_3$ | 4-Cl | 2-Cl | H | 2 | (4) | 162-163 |
| 58 | 5-$CH_3$ | H | H | H | 2 | (4) | 155-156 |
| 59 | 4-Cl | H | 3-$CH_3$ | H | 2 | (4) | 181-182 |
| 60 | 4-Cl | H | 2-$CH_3$ | H | 2 | (4) | 188-189 |
| 61 | 6-$CH_3$ | 4-Cl | H | H | 2 | (4) | 157-158 |
| 62 | 4-Cl | H | 4-Cl | H | 2 | (4) | 162-163 |
| 63 | 4-Cl | H | 3-Cl | H | 2 | (4) | 177-178 |
| 64 | 5-$CH_3$ | 4-Cl | H | H | 2 | (4) | 161-162 |
| 65 | H | H | H | H | 2 | (4) | 145-146 |
| 66 | 4-$CH_3$ | H | H | H | 1 | (2) | 194-196 |
| 67 | 4-Cl | H | 2-F | H | 1 | (2) | 180-182 |
| 68 | 4-Cl | H | 2-$CH_3$ | 5-$CH_3$ | 1 | (2) | 164-166 |
| 69 | H | H | H | H | 1 | (2) | 182-184 |
| 70 | 4-$CH_3$ | H | 2-Cl | H | 1 | (2) | 181-183 |
| 71 | 4-CL | H | H | H | 1 | (2) | 207-208 |
| 72 | 4-Cl | H | 2-Cl | H | 1 | (2) | 113-114 |
| 73 | 5-$OCH_3$ | H | H | H | 1 | (4) | 172-174 |
| 74 | 6-$CH_3$ | 4-$CH_3$ | H | H | 1 | (4) | 162-164 |

(*) : (2) représente 1_H_-imidazolyle-2

(4) représente 1_H_-imidazolyle-4

Les composés de l'invention ont été testés en pharmacologie. Leur activité analgésique a été montrée dans le test de Koster et al ("writhing test" à l'acide acétique chez la souris), Fed. Proc., 18, 412, 1959.

On administre par voie orale, aux souris à jeun, le composé à tester en solution dans du Tween 80 à 1%, à raison de 0,2 ml par 20 g de poids corporel ; au bout de 30 mn on administre l'acide acétique (en solution à 0,6% dans un mélange carboxy-méthyl-cellulose et tween 80, à raison de 10 ml par kg de poids corporel) par voie intrapéritonéale. On note le nombre total de contorsions pendant 15 mn.

On détermine le pourcentage de protection par rapport à un lot témoin et on détermine la DA 50 par voie graphique (dose qui protège 50% d'animaux).

La DA 50 des composés de l'invention va de 2 à 200 mg/kg p.o.

Les composés présentent également une activité antidépressive, une activité antidyskinétique, une activité antiulcéreuse et ils diminuent la sécrétion de l'acide gastrique.

L'activité antiulcéreuse des composés a été montrée dans les tests de l'ulcère de stress et de l'ulcère induit par la phénylbutazone.

Ulcère de stress

La technique utilisée est celle de Senay et Levine , Proc. Soc. Exp. Biol. 1967, 124, 1221-1223, Peptic Ulcers. Edited by C.J. PFEIFER. p. 92-97, sur des rats Wistar femelles pesant 180-210 g, à jeun depuis 20 heures, répartis en blocs randomisés.

Les animaux sont mis en contention dans des boîtes cylindriques de 20 cm x 5 cm et placés dans une chambre froide dont la température est maintenue à 2-4°C.

Les composés à étudier sont administrés p.o. à raison de 10, 30 et 100 mg/kg immédiatement avant la mise en contention, les rats témoins recevant seulement le placébo.

2 heures plus tard, les animaux sont sacrifiés par inhalation de chloroforme.

Les estomacs sont prélevés et le degré d'ulcération est noté.

Les composés de l'invention diminuent significativement ($\leqslant$-92%) les ulcères de stress.

Ulcère induit par la phénylbutazone

Le test est effectué sur des rats Wistar femelles pesant 180-210 g à jeun depuis 20 heures, répartis en blocs randomisés.

Les ulcères sont provoqués par l'administration orale de phénylbutazone en solution mole à mole avec NaOH, à la dose de 200 mg/kg.

Les composés à étudier sont administrés p.o. à raison de 10, 30 ou 100 mg/kg, 30 minutes avant l'ingestion de phénylbutazone, les animaux témoins ne recevant que le placébo.

Deux heures après l'administration de l'agent ulcérigène, les animaux sont sacrifiés par inhalation de chloroforme.

Les estomacs sont prélevés et le degré d'ulcération est noté.

Les composés de l'invention diminuent de manière significative (jusqu'à 100 %) les ulcères induits par la phénylbutazone.

La toxicité aiguë des composés a été déterminée. La DL 50 par voie orale varie de 70 à plus de 1000 mg/kg.

Les composés de l'invention possèdent des propriétés antidépressives, analgésiques et antiulcéreuses et peuvent donc être utilisés pour le traitement de la dépression, de la douleur d'origines diverses (par exemple post-chirurgicale, dentaire, migraines...) et des ulcères gastriques, duodénaux ou gastroduodénaux.

Les composés de l'invention peuvent être administrés par voie orale ou parentérale, sous la forme de compositions pharmaceutiques contenant la substance active en association avec tout excipient approprié ; par exemple sous la forme de comprimés, dragées, gélules, capsules ou de solutions ou de suspensions

buvables ou injectables.

La posologie quotidienne peut aller de 10 à 2000 mg.

0133061

Revendications pour les états contractants : BE, CH, DE, FR, GB, IT, LI, LU, NL, SE.

1. Diphénylazométhines répondant à la formule

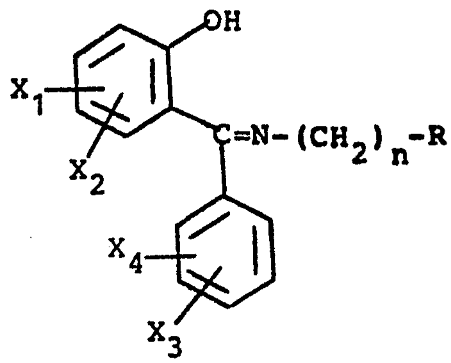

(I)

dans laquelle

$X_1$, $X_2$, $X_3$ et $X_4$ représentent chacun, indépendamment l'un de l'autre, un atome d'hydrogène ou d'halogène ou un groupe nitro, cyano, hydroxy, méthoxy, alkyle en $C_1$-$C_3$, allyle ou phényle, et

soit R représente un radical 1H-imidazolyle-2 et n est égal à 1,

soit R représente un radical 1H-imidazolyle-4 et n est égal à 1 ou 2.

2. Composés selon la revendication 1, dans lesquels $X_1$ et $X_3$ représentent, indépendamment l'un de l'autre un atome d'hydrogène, de fluor, de chlore ou de brome et $X_2$ représente un atome d'hydrogène ou le radical méthyle ou allyle.

3. Le chloro-4 [(chloro-2 phényl){[(1H-imidazolyl-4)méthyl]imino} méthyl]-2 phénol.

4. Procédé de préparation des composés selon la revendication 1, procédé caractérisé en ce que l'on fait réagir une benzophénone (II)

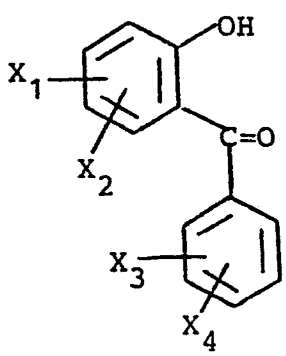

(II)

avec un composé $H_2N(CH_2)_nR$ (III) éventuellement sous la forme de chlorhydrate.

5. Médicament caractérisé en ce qu'il contient un composé tel que spécifié dans l'une quelconque des revendications 1 à 3.

6. Composition pharmaceutique caractérisée en ce qu'elle contient un composé tel que spécifié dans l'une quelconque des revendications 1 à 3 en association avec tout excipient approprié.

Revendications pour l'état contractant : AT.

1. Procédé de préparation de composés répondant à la formule

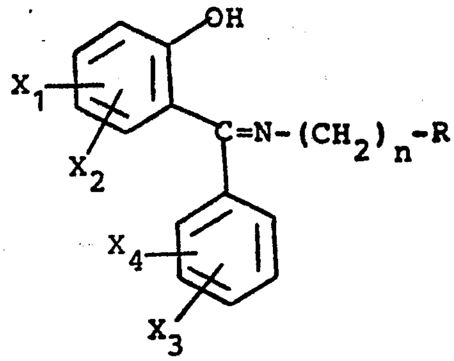

(I)

dans laquelle

$X_1$, $X_2$, $x_3$ et $X_4$ représentent chacun, indépendamment l'un de l'autre, un atome d'hydrogène ou d'halogène ou un groupe nitro, cyano,hydroxy, méthoxy, alkyle en $C_1$-$C_3$, allyle ou phényle, et

soit R représente un radical 1H-imidazolyle-2 et n est égal à 1,

soit R représente un radical 1H-imidazolyle-4 et n est égal à 1 ou 2,

procédé caractérisé en ce que l'on fait réagir une benzophénone (II)

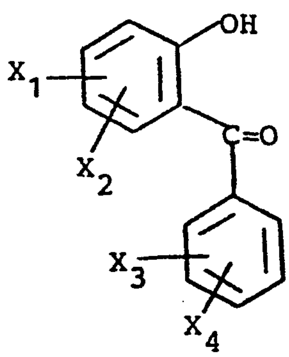

(II)

avec un composé $H_2N(CH_2)_nR$ (III) éventuellement sous la forme de chlorhydrate.

2. Procédé selon la revendication 1, caractérisé en ce qu'on utilise une benzophénone de formule II dans laquelle $X_1$ et $X_3$ représentent, indépendamment l'un de l'autre un atome d'hydrogène, de fluor, de chlore ou de brome et $X_2$ représente un atome d'hydrogène ou le radical méthyle ou allyle.

3. Procédé selon la revendication 1, caractérisé en ce qu'on prépare le chloro-4[(chloro-2 phényl) {[(1H-imidazolyl-4) méthyl]imino}méthyl]-2 phénol.

Office européen des brevets

**RAPPORT DE RECHERCHE EUROPEENNE**

0133061

Numéro de la demande

EP 84 40 1254

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 081 324 (FARMOS)<br>----- | | C 07 D 233/64<br>A 61 K 31/415 |
| | | | **DOMAINES TECHNIQUES RECHERCHES (Int. Cl.4)** |
| | | | C 07 D 233/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 15-10-1984 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82